# EUROPÄISCHE PATENTANMELDUNG

(11) **EP 3 348 190 A1**
(43) Veröffentlichungstag der Anmeldung: **18.07.2018**
(21) Anmeldenummer: 17151486.2
(22) Anmeldetag: 13.01.2017
(51) Int. Cl.: A61B 5/055

(54) **MRT-VORRICHTUNG ZUR VERMESSUNG EINES KOPFBEREICHS**

(71) Anmelder: Sirona Dental Systems GmbH, 64625 Bensheim (DE)
(72) Erfinder: Hell, Erich, 66557 Wustweiler (DE); Ulrici, Johannes, 64285 Darmstadt (DE); Rasche, Volker, 89155 Erbach (DE)
(74) Vertreter: Sommer, Peter

(57) **Zusammenfassung**

Die Erfindung betrifft eine MRT-Vorrichtung (1) zur Vermessung eines Kopfbereichs (2) eines Patienten (4), umfassend mindestens eine Hauptmagnetfeldeinheit (5) und mindestens eine Patientenöffnung (6). Die MRT-Vorrichtung (1) ist dabei schräg angeordnet und eine Mittelachse (7) der MRT-Vorrichtung (1) weist relativ zu einer Erdgravitationskraftrichtung (8) einen Winkel (9) zwischen 20° und 75° auf, wobei die MRT-Vorrichtung (1) einen Patientensitz (10) aufweist, wobei der Patientensitz (10) Verstellmittel (13) aufweist, wobei der auf dem Patientensitz (10) positionierte Patient (4) mittels der Verstellmittel (13) entlang einer Fahrkurve (14) in die MRT-Vorrichtung (1) zumindest teilweise hineingefahren wird, bis der Kopfbereich (2) des Patienten (4) in einem Aufnahmebereich (17) der MRT-Vorrichtung (1) angeordnet ist und der Patientensitz (10) in eine Aufnahmeposition (16) gebracht wird, wobei die Fahrkurve (14) einen Winkel relativ zu der Mittelachse (7) der MRT-Vorrichtung (1) aufweist, der innerhalb eines Toleranzwinkelbereichs zwischen +10° und -10° relativ zu der Mittelachse (7) der MRT-Vorrichtung (1) liegt.

## Beschreibung

### Technisches Gebiet

Die Erfindung betrifft eine MRT-Vorrichtung zur Vermessung zumindest eines Kopfbereichs eines Patienten, umfassend mindestens eine Hauptmagnetfeldeinheit und mindestens eine Patientenöffnung.

### Stand der Technik

Aus dem Stand der Technik sind mehrere MRT-Vorrichtungen zur Vermessung eines Patienten bekannt.

DE 10 2009 027119 B4 offenbart ein MRT-System zur bildgebenden Erfassung eines Kopfbereichs mit einem Permanentmagneten, einer Gradientenspule und mindestens einer Hochfrequenzspule, wobei eine Magnetfeldeinheit an einem vertikal angeordneten Stativ in der Höhe verstellt werden kann. Zusätzlich kann die Magnetfeldeinheit bis zu einem Winkel von 45 ° relativ zur Längsachse des Stativs geschwenkt werden. Der Patient nimmt während der Vermessung eine sitzende Position ein, wobei der Kopf des Patienten durch eine Stirnstütze und Ohroliven relativ zum Stativ positioniert wird.

Bei diesem MRT-System wird der Patient auf eine aufwändige Art und Weise relativ zur MRT-Vorrichtung positioniert, indem der Patient in einer zylinderförmigen Patientenöffnung positioniert wird. Dabei werden ein höhenverstellbarer Hocker und das vertikal verstellbare Stativ des MRT-Systems manuell so eingestellt, dass der Kopf des Patienten vermessen werden kann.

DE 197 34 138 B2 offenbart eine MRT-Vorrichtung, umfassend eine Gradienten-Spulenanordnung und eine MRT-Hauptspule.

US 2013/0023418 A1 offenbart eine MRT-Vorrichtung mit einem Kühlungssystem aus einer ersten Stufe und einer zweiten Stufe.

Die Aufgabe der vorliegenden Erfindung besteht also darin, eine MRT-Vorrichtung bereitzustellen, die eine bequeme und einfache Positionierung des Patienten relativ zur MRT-Vorrichtung ermöglicht wird.

### Darstellung der Erfindung

Die Erfindung betrifft eine MRT-Vorrichtung zur Vermessung eines Kopfbereichs eines Patienten, umfassend mindestens eine Hauptmagnetfeldeinheit und mindestens eine Patientenöffnung. Die MRT-Vorrichtung ist dabei schräg angeordnet, wobei eine Mittelachse der MRT-Vorrichtung relativ zu einer Gravitationskraftrichtung einen Winkel zwischen 20° und 75° aufweist, wobei die MRT-Vorrichtung einen Patientensitz aufweist, wobei der Patientensitz Verstellmittel aufweist. Der auf dem Patientensitz positionierte Patient wird mittels der Verstellmittel entlang einer Fahrkurve in die MRT-Vorrichtung zumindest teilweise hineingefahren, bis der Kopfbereich des Patienten in einem Aufnahmebereich der MRT-Vorrichtung angeordnet ist und der Patientensitz in eine Aufnahmeposition gebracht wird, wobei die Fahrkurve einen Winkel relativ zu einer Mittelachse der MRT-Vorrichtung aufweist, der innerhalb eines Toleranzwinkelbereichs zwischen +10° und -10° relativ zu der Mittelachse der MRT-Vorrichtung liegt.

Die MRT-Vorrichtung (Magnetresonanztomographie-Vorrichtung) ist eine herkömmliche MRT-Vorrichtung zur Vermessung eines Kopfes. Die MRT-Vorrichtung weist dabei ein Messvolumen bzw. einen Aufnahmebereich auf, wobei das Objektvolumen des Objekts innerhalb des Messvolumens angeordnet wird, um das Objekt, nämlich den Kopfbereich zu vermessen.

Die Hauptmagnetfeldeinheit erzeugt das Hauptmagnetfeld und kann beispielsweise aus mindestens einem Dauermagneten, mindestens einer Magnetspule oder mindestens einer supraleitenden Magnetspule bestehen.

Die Patientenöffnung kann beliebig geformt sein und beispielsweise eine kreisförmige Form, eine elliptische Form oder eine rechteckige Form aufweisen. Die Patientenöffnung muss so groß gestaltet sein, dass insbesondere der Kopfbereich des Patienten in den Messbereich der MRT-Vorrichtung hineinpasst.

Die Mittelachse der MRT-Vorrichtung kann beispielsweise mit einer Symmetrieachse der Patientenöffnung übereinstimmen. Die Mittelachse der MRT-Vorrichtung kann auch mit der Symmetrieachse des Hauptmagnetfeldes der Hauptmagnetfeldeinheit übereinstimmen bzw. parallel zu der Symmetrieachse des Hauptmagnetfeldes ausgerichtet sein.

Der zu vermessende Kopfbereich kann beispielsweise einen Oberkiefer, einen Unterkiefer und/oder mindestens ein Kiefergelenk umfassen. Die Verstellmittel des Patientensitzes erlauben die Verstellung entlang der Fahrkurve, wobei die Verstellmittel beispielsweise als eine Führungsschiene ausgeführt sein können. Die Fahrkurve kann eine gerade oder eine gekrümmte Fahrkurve sein. In der Aufnahmeposition wird der aufzunehmende Kopfbereich innerhalb des Aufnahmebereichs bzw. des Messvolumens der MRT-Vorrichtung positioniert.

Die Fahrkurve ist innerhalb des Toleranzbereichs nahezu parallel zur Mittelachse der MRT-Vorrichtung, so dass ein bequemes Hineinfahren des Patienten in die Patientenöffnung ermöglicht wird.

Die MRT-Vorrichtung ist schräg angeordnet, wobei die Mittelachse der MRT-Vorrichtung relativ zu der Richtung der Erdgravitation ein Winkel zwischen 20° und 75° aufweist. Der Patient wird also in eine sitzende Position gebracht und schräg in die MRT-Vorrichtung hineingefahren.

Ein Vorteil der vorliegenden MRT-Vorrichtung besteht darin, dass ein schräg sitzender Patient auf eine komfortable Art und Weise auf einem Patientensitz positioniert werden kann und vollautomatisch in die MRT-Vorrichtung hineingefahren werden kann. Denn der Patient kann in einer nach hinten gelehnten, sitzenden Position auf einem Patientensitz positioniert werden und in die MRT-Vorrichtung hineingefahren werden. Durch die schräge Anordnung der MRT-Anordnung werden also die Knie nicht so stark angewinkelt wie in einer aufrecht sitzenden Patientenposition, so dass die Kollision einer unteren Fläche der MRT-Vorrichtung mit den Oberschenkeln oder den Knien des Patienten beim Hineinfahren verhindert wird.

Vorteilhafterweise kann an der MRT-Vorrichtung ein Patientensitz angebracht sein, wobei der Patientensitz in der Aufnahmeposition einen Winkel zwischen einer Rückenlehne und einer Sitzfläche zwischen 100° und 170° aufweist.

Dadurch wird eine bequeme Positionierung des Patienten auf dem Patientensitz ermöglicht.

Vorteilhafterweise kann der Patientensitz eine verstellbare Rückenlehne aufweisen, wobei die Rückenlehne zwischen einer Einstiegsposition und der Aufnahmeposition verstellbar ist, wobei in der Einstiegsposition der Winkel zwischen der Rückenlehne und der Sitzfläche zwischen 80° und 110° beträgt und in der Aufnahmeposition der Winkel zwischen der Rückenlehne und der Sitzfläche zwischen 110° und 170° beträgt. Der Patient wird also im ersten Schritt auf dem Patientensitz in der Einstiegsposition positioniert, wobei der Patientensitz im zweiten Schritt in die Aufnahmeposition verstellt wird, so dass die Rückenlehne nach hinten gelehnt wird und der Patient in die Schräglage gebracht wird.

Vorteilhafterweise kann die Fahrkurve des Patientensitzes parallel zu der Mittelachse der MRT-Vorrichtung angeordnet sein.

Durch die parallele Anordnung wird ein bequemes und sicheres Hineinfahren des Patienten ermöglicht.

Vorteilhafterweise können die Verstellmittel mechanisch durch einen Benutzer verstellt werden, um den Patientensitz in die Aufnahmeposition zu bringen.

Dadurch wird auf eine einfache Art und Weise das Hineinfahren des Patienten ohne eines Antriebsmittels ermöglicht.

Vorteilhafterweise können die Verstellmittel des Patientensitzes durch mindestens ein Antriebsmittel angetrieben werden, welches mittels einer Steuerungseinheit angesteuert wird, um den Patientensitz in die Aufnahmeposition zu bringen.

Dadurch wird ein vollautomatisches Hineinfahren des Patienten in die Aufnahmeposition ermöglicht, wobei das Antriebsmittel mittels der Steuerungseinheit entsprechend angesteuert wird. Das Antriebsmittel kann beispielsweise ein Elektromotor, ein pneumatischer Antrieb oder ein hydraulischer Antrieb sein. Die Steuerungseinheit kann ein Mikrocomputer oder ein Mikrochip sein.

Vorteilhafterweise kann die Mittelachse der MRT-Vorrichtung parallel zu einer Symmetrieachse eines Hauptmagnetfeldes der Hauptmagnetfeldeinheit oder parallel zu einer Symmetrieachse der Patientenöffnung ausgerichtet sein.

Dadurch wird die Mittelachse eindeutig vorgegeben.

Vorteilhafterweise kann der Patientensitz mindestens eine Armlehne aufweisen.

Dadurch wird eine bequeme Positionierung des Patienten, insbesondere der Arme des Patienten, ermöglicht.

Vorteilhafterweise kann die Armlehne zwischen einer Einstiegsposition und einer Aufnahmeposition verstellbar sein, wobei der Abstand zwischen der Armlehne und der Sitzfläche in der Einstiegsposition größer ist als in der Aufnahmeposition.

Dadurch wird die Höhe der Armlehne entsprechend in Abhängigkeit von der Schräglage der Rückenlehne angepasst.

Vorteilhafterweise kann an der Armlehne oder an der Sitzfläche des Patientensitzes ein Kommunikationselement angeordnet sein.

Dadurch wird dem Patienten auf eine einfache Art und Weise ermöglicht, das Kommunikationselement beim Hineinfahren des Patientensitzes und nach dem Erreichen der Aufnahmeposition zu bedienen. Das Kommunikationselement ermöglicht es dem Patienten ein akustisches oder visuelles Signal beispielsweise bei Unwohlsein abzugeben.

Vorteilhafterweise kann die MRT-Vorrichtung eine Detektorvorrichtung aufweisen, die eine Lage des Patientensitzes relativ zu der Hauptmagnetfeldeinheit bestimmt.

Dadurch ermöglicht die Detektorvorrichtung die Lage des Patientensitzes zu bestimmen, so dass die Länge der noch zu verfahrenden Fahrkurve bis zum Erreichen der Aufnahmeposition ermittelt werden kann.

Vorteilhafterweise kann die Detektorvorrichtung eine Laser-Vorrichtung sein, die auf einem Triangulation-Messverfahren, einem Phasenverschiebung-Verfahren oder einem Laufzeit-Verfahren beruht, wobei die Laser-Vorrichtung einen Laser, einen Reflektor und einen Sensor umfasst, wobei der Laser eine definierte Lage relativ zur Hauptmagnetfeldeinheit aufweist, wobei der Reflektor am Patientensitz angeordnet ist.

Durch die Verwendung der Laser-Vorrichtung wird eine präzise Ermittlung der Lage ermöglicht.

Vorteilhafterweise kann die Detektorvorrichtung eine Seilzug-Vorrichtung umfassend einen Seilzug-Potentiometer und eine Seilöse sein, wobei der Seilzug-Potentiometer eine definierte Lage relativ zur Hauptmagnetfeldeinheit aufweist und die Seilöse am Patientensitz angeordnet ist.

Durch die Verwendung der Seilzug-Vorrichtung wird die Lage zuverlässig ermittelt.

Vorteilhafterweise kann die Detektorvorrichtung einen Sensor, wie eine Lichtschranke, und eine Schaltfahne aufweisen, wobei der Sensor eine definierte Lage relativ zur Hauptmagnetfeldeinheit aufweist und die Schaltfahne am Patientensitz angeordnet ist, wobei der Sensor so angeordnet ist, dass beim Hineinfahren des Patientensitzes oder beim Erreichen der Aufnahmeposition der Sensor ein Signal erzeugt, sobald die Schaltfahne den Sensor durchfährt.

Durch die Verwendung der Lichtschranke wird die Lage zuverlässig beim Durchfahren der Lichtschranke ermittelt.

Die Erfindung betrifft weiterhin ein Verfahren zur Positionierung eines Patienten relativ zu der oben genannten MRT-Vorrichtung. Der Patient wird auf dem Patientensitz positioniert und der Patientensitz wird in die Aufnahmeposition gebracht, so dass der aufzunehmende Kopfbereich des Patienten in den Aufnahmebereich der MRT-Vorrichtung gebracht wird.

Ein Vorteil dieses Verfahrens besteht darin, dass der Schrägsitzende Patient auf eine komfortable und zeitsparende Art und Weise in die MRT-Vorrichtung bis zum Erreichen der Aufnahmeposition hineingefahren werden kann.

Vorteilhafterweise kann der Patientensitz eine verstellbare Rückenlehne aufweisen, wobei die Rückenlehne zwischen einer Einstiegsposition und der Aufnahmeposition verstellbar ist, wobei der Winkel in der Einstiegsposition kleiner als der Winkel in der Aufnahmeposition ist, wobei in der Einstiegsposition der Winkel zwischen der Rückenlehne und der Sitzfläche zwischen 80° und 150° beträgt und in der Aufnahmeposition der Winkel zwischen der Rückenlehne und der Sitzfläche zwischen 110° und 170° beträgt.

Der Patient wird also im ersten Schritt auf dem Patientensitz in der Einstiegsposition positioniert, wobei im zweiten Schritt der Patientensitz in die Aufnahmeposition gebracht wird, wobei die Rückenlehne nach hinten gelehnt wird und der Patient in die Schräglage gebracht wird.

Vorteilhafterweise können die Verstellmittel des Patientensitzes mechanisch durch einen Benutzer verstellt werden, um den Patientensitz in die Aufnahmeposition zu bringen.

Dadurch wird das Hineinfahren des Patienten ohne Antriebsmittel ermöglicht.

Vorteilhafterweise können die Verstellmittel des Patientensitzes durch mindestens ein Antriebsmittel angetrieben werden und mittels einer Steuerungseinheit angesteuert werden, um den Patientensitz in die Aufnahmeposition zu bringen.

Dadurch wird ein vollautomatisches Hineinfahren des Patienten bis zur Aufnahmeposition ermöglicht.

### Kurzbeschreibung der Zeichnungen

Die Erfindung wird anhand der Zeichnungen erläutert. Es zeigt, die
- Fig. 1: eine Skizze einer MRT-Vorrichtung, die
- Fig. 2: eine Skizze des Patientensitzes mit verstellbarer Rückenlehne.

### Ausführungsbeispiele

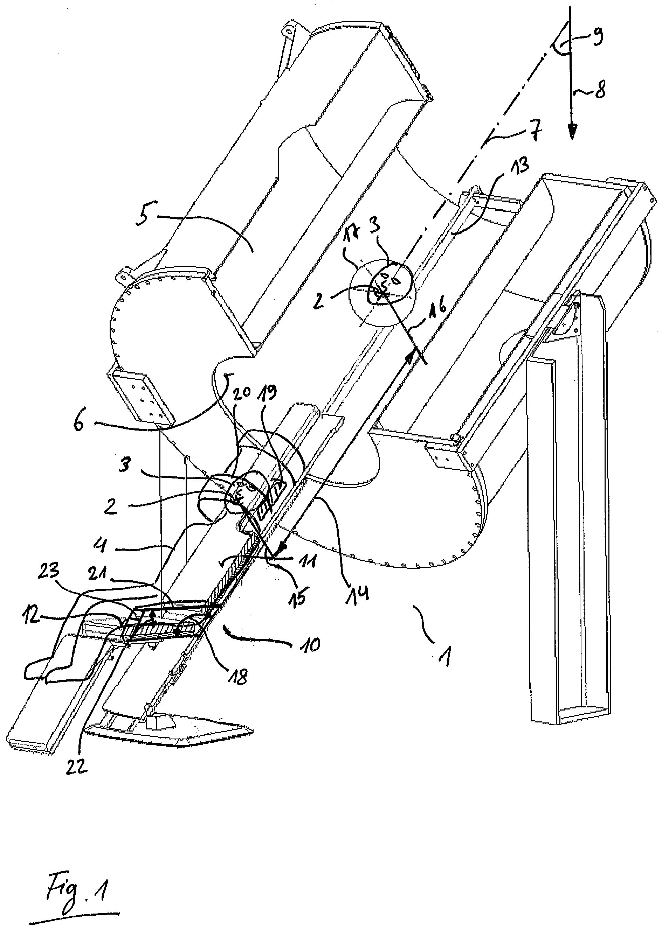

Die Fig. 1 zeigt eine Skizze einer MRT-Vorrichtung 1 zur Vermessung eines Kopfbereichs 2 eines Kopfes 3 eines Patienten 4, umfassend mindestens eine Hauptmagnetfeldeinheit 5 und mindestens eine Patientenöffnung 6. Der aufzunehmende Kopfbereich 2 ist durch ein Kreuz angedeutet und kann beispielsweise einen Unterkiefer, einen Oberkiefer, ein Kiefergelenk oder auch nur Teile davon umfassen. Die MRT-Vorrichtung 1 ist schräg angeordnet, wobei eine Mittelachse 7 der MRT-Vorrichtung 1 relativ zu einer Erdgravitationskraftrichtung 8 einen Winkel 9 zwischen 20° und 75°, im vorliegenden Fall 45°, aufweist. An der MRT-Vorrichtung 1 ist ein Patientensitz 10 angebracht, wobei der Patientensitz 10 eine Rückenlehne 11 und eine Sitzfläche 12 aufweist. Der Patientensitz 10 wird mittels eines Verstellmittels 13, wie einer Führungsschiene, entlang einer Fahrkurve 14 ausgehend von einer Ausgangsposition 15 in eine Aufnahmeposition 16 hineingefahren. Die Ausgangsposition 15 und die Aufnahmeposition 16 sind durch Striche angeordnet. Die Fahrkurve 14 ist durch einen graden Pfeil angedeutet. In der Aufnahmeposition 16 ist der aufzunehmende Kopfbereich 2 des Kopfes 3 innerhalb eines Aufnahmebereichs 17 der MRT-Vorrichtung angeordnet, so dass der Kopfbereich in der Aufnahmeposition 16 vermessen werden kann. Im vorliegenden Fall ist der Aufnahmebereich 17 durch eine Kugel dargestellt. Der Patientensitz weist einen Winkel 18 zwischen der Rückenlehne 11 und der Sitzfläche 12 zwischen 100° und 170°, im vorliegenden Fall 135°, auf. Vor dem Hineinfahren wird eine Kopfhalterung 19 und eine Hochfrequenzspule 20 relativ zum Kopf 3 des Patienten 4 positioniert. Der Patientensitz 10 weist darüber hinaus eine Armlehne 21 auf, wobei ein Abstand zwischen der Armlehne 21 und der Sitzfläche 12 verstellbar ist. An der Armlehne 21 ist ein Kommunikationselement angebracht, so dass der Patient 4 beim Hineinfahren des Patientensitzes 10 in die Aufnahmeposition 16 und nach dem Hineinfahren ein Signal, beispielsweise bei Unwohlsein, abgeben kann.

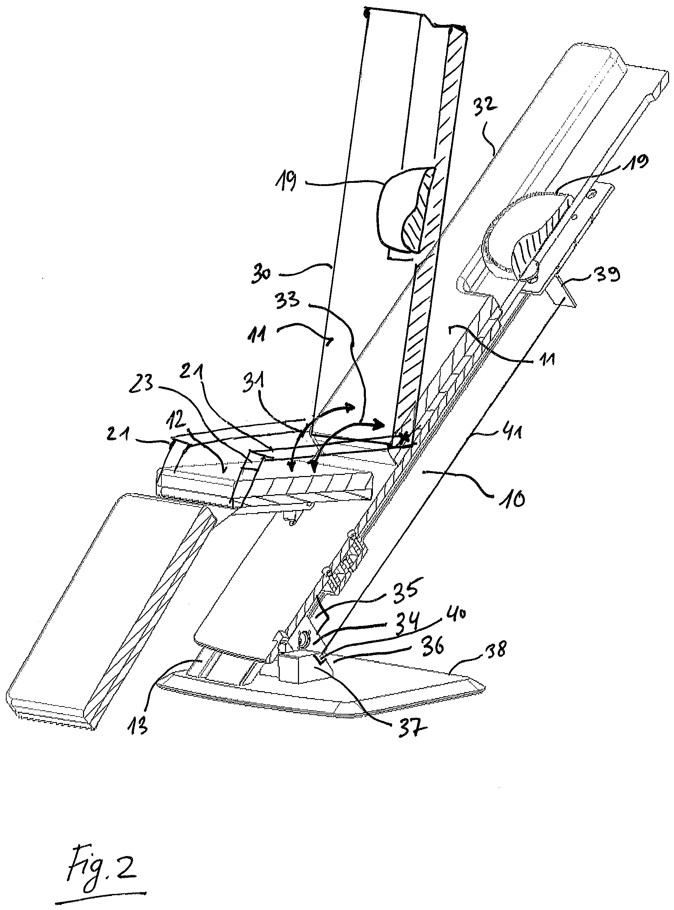

Die Fig. 2 zeigt den Patientensitz 10 aus Fig. 1, wobei in einer Einstiegsposition 30 ein Winkel 31 zwischen der Rückenlehne 11 und der Sitzfläche 12 zwischen 80° und 150°, im vorliegenden Fall 95°, beträgt, wobei in einer Aufnahmeposition 32 ein zweiter Winkel 33 zwischen der Rückenlehne 11 in der Aufnahmeposition 32 und der Sitzfläche 12 zwischen 110° und 170°, im vorliegenden Fall 135° beträgt. Das Hineinfahren des Patientensitzes erfolgt mittels des Verstellmittels 13, wie einer Führungsschiene, angetrieben durch ein Antriebsmittel 34, wobei das Antriebsmittel 34 durch eine Steuerungseinheit 35 angesteuert wird, um den Patientensitz 10 in die Aufnahmeposition zu bringen. Mittels einer Detektorvorrichtung 36 wird eine Lage des Patientensitzes relativ zu der Hauptmagnetfeldeinheit 5 aus Fig. 1 ermittelt. Im vorliegenden Fall ist die Detektorvorrichtung einer Laser-Vorrichtung, umfassend einen Laser 37, der an einem Stativ 38 angebracht ist, einem Reflektor 39, der an der Kopfhalterung 19 angebracht ist und einen Sensor 40. Der Laser 37 erzeugt einen Laserstrahl 41, der vom Reflektor reflektiert wird und mittels des Sensors 40 detektiert wird. Unter Verwendung eines Triangulations-Messverfahrens eines Phasenverschiebung-Verfahrens oder eines Laufzeit-Verfahrens kann dann der Abstand zwischen dem Laser 37 und dem Reflektor 39 bestimmt werden. Ausgehend von diesem Abstand kann dann die Lage der Kopfhalterung 19 und damit des Patientenstuhls 10 relativ zur Hauptfeldeinheit aus Fig. 1 bestimmt werden.

### Bezugszeichen

- 1: MRT-Vorrichtung
- 2: Kopfbereich
- 3: Kopf
- 4: Patient
- 5: Hauptmagnetfeld
- 6: Patientenöffnung
- 7: Mittelachse
- 8: Erdgravitationskraftrichtung
- 9: Winkel
- 10: Patientensitz
- 11: Rückenlehne
- 12: Sitzfläche
- 13: Verstellmittel
- 14: Fahrkurve
- 15: Ausgangsposition
- 16: Aufnahmeposition
- 17: Aufnahmebereich
- 18: Winkel
- 19: Kopfhalterung
- 20: Hochfrequenzspule
- 21: Armlehne
- 30: Einstiegsposition
- 31: Winkel
- 32: Aufnahmeposition
- 33: Zweiter Winkel
- 34: Antriebsmittel
- 35: Steuerungseinheit
- 36: Detektorvorrichtung
- 37: Laser
- 38: Stativ
- 39: Reflektor
- 40: Sensor
- 41: Laserstrahl

## Patentansprüche

1. MRT-Vorrichtung (1) zur Vermessung eines Kopfbereichs (2) eines Patienten (4), umfassend mindestens eine Hauptmagnetfeldeinheit (5) und mindestens eine Patientenöffnung (6), **dadurch gekennzeichnet, dass** die MRT-Vorrichtung (1) schräg angeordnet ist und eine Mittelachse (7) der MRT-Vorrichtung (1) relativ zu einer Erdgravitationskraftrichtung (8) einen Winkel (9) zwischen 20° und 75° aufweist, wobei die MRT-Vorrichtung (1) einen Patientensitz (10) aufweist, wobei der Patientensitz (10) Verstellmittel (13) aufweist, wobei der auf dem Patientensitz (10) positionierte Patient (4) mittels der Verstellmittel (13) entlang einer Fahrkurve (14) in die MRT-Vorrichtung (1) zumindest teilweise hineingefahren wird, bis der Kopfbereich (2) des Patienten (4) in einem Aufnahmebereich (17) der MRT-Vorrichtung (1) angeordnet ist und der Patientensitz (10) in eine Aufnahmeposition (16) gebracht wird, wobei die Fahrkurve (14) einen Winkel relativ zu der Mittelachse (7) der MRT-Vorrichtung (1) aufweist, der innerhalb eines Toleranzwinkelbereichs zwischen +10° und -10° relativ zu der Mittelachse (7) der MRT-Vorrichtung (1) liegt.

2. MRT-Vorrichtung (1) nach Anspruch 1, **dadurch gekennzeichnet, dass** an der MRT-Vorrichtung (1) ein Patientensitz (10) angebracht ist, wobei der Patientensitz (10) in der Aufnahmeposition (16) einen Winkel (33) zwischen einer Rückenlehne (11) und einer Sitzfläche (12) zwischen 100° und 170° aufweist.

3. MRT-Vorrichtung (1) nach Anspruch 1 oder 2, **dadurch gekennzeichnet, dass** der Patientensitz (10) eine verstellbare Rückenlehne (11) aufweist, wobei die Rückenlehne (11) zwischen einer Einstiegsposition (30) und der Aufnahmeposition (32) verstellbar ist, wobei der Winkel (31) in der Einstiegsposition (30) kleiner als der Winkel (33) in der Aufnahmeposition (32) ist, wobei in der Einstiegsposition (30) der Winkel (31) zwischen der Rückenlehne (11) und der Sitzfläche (12) zwischen 80° und 150° beträgt und in der Aufnahmeposition (32) der Winkel (33) zwischen der Rückenlehne (11) und der Sitzfläche (12) zwischen 110° und 170° beträgt.

4. MRT-Vorrichtung (1) nach einem der Ansprüche 1 bis 3, **dadurch gekennzeichnet, dass** die Fahrkurve (14) des Patientensitzes (10) parallel zu der Mittelachse (7) der MRT-Vorrichtung (1) angeordnet ist.

5. MRT-Vorrichtung (1) nach einem der Ansprüche 1 bis 4, **dadurch gekennzeichnet, dass** die Verstellmittel (13) mechanisch durch einen Benutzer verstellt werden, um den Patientensitz (10) in die Aufnahmeposition (16) zu bringen.

6. MRT-Vorrichtung (1) nach einem der Ansprüche 1 bis 5, **dadurch gekennzeichnet, dass** die Verstellmittel (13) des Patientensitzes (10) durch mindestens ein Antriebsmittel (34) angetrieben werden, welches mittels einer Steuerungseinheit (35) angesteuert wird, um den Patientensitz (10) in die Aufnahmeposition (16) zu bringen.

7. MRT-Vorrichtung (1) nach einem der Ansprüche 1 bis 6, **dadurch gekennzeichnet, dass** die Mittelachse (7) der MRT-Vorrichtung (1) parallel zu einer Symmetrieachse eines Hauptmagnetfeldes der Hauptmagnetfeldeinheit oder parallel zu einer Symmetrieachse der Patientenöffnung (6) ausgerichtet ist oder mit dieser übereinstimmt.

8. MRT-Vorrichtung (1) nach einem der Ansprüche 1 bis 7, **dadurch gekennzeichnet, dass** der Patientensitz (10) mindestens eine Armlehne (21) aufweist.

9. MRT-Vorrichtung (1) nach Anspruch 8, **dadurch gekennzeichnet, dass** die Armlehne (21) zwischen einer Einstiegsposition (30) und einer Aufnahmeposition (32) verstellbar ist, wobei der Abstand (22) zwischen der Armlehne (21) und der Sitzfläche (12) in der Einstiegsposition (30) größer ist als in der Aufnahmeposition (32, 16).

10. MRT-Vorrichtung (1) nach Anspruch 8 oder 9, **dadurch gekennzeichnet, dass** an der Armlehne (21) oder an der Sitzfläche (12) des Patientensitzes (10) ein Kommunikationselement (23) angeordnet ist.

11. Verfahren zur Positionierung eines Patienten (4) relativ zu einer MRT-Vorrichtung (1) nach einem der Ansprüche 1 bis 7, **dadurch gekennzeichnet, dass** der Patient auf dem Patientensitz (10) positioniert wird und der Patientensitz (10) in die Aufnahmeposition (16) gebracht wird, so dass der aufzunehmende Kopfbereich (2) des Patienten (4) in den Aufnahmebereich (17) der MRT-Vorrichtung (1) gebracht wird.

12. Verfahren nach Anspruch 11, **dadurch gekennzeichnet, dass** der Patientensitz (10) eine verstellbare Rückenlehne (11) aufweist, wobei die Rückenlehne (11) zwischen einer Einstiegsposition (30) und der Aufnahmeposition (32) verstellbar ist, wobei der Winkel (31) in der Einstiegsposition (30) kleiner als der Winkel (33) in der Aufnahmeposition (32) ist, wobei in der Einstiegsposition (30) der Winkel (31) zwischen der Rückenlehne (11) und der Sitzfläche (12) zwischen 80° und 150° beträgt und in der Aufnahmeposition (32) der Winkel (33) zwischen der Rückenlehne (11) und der Sitzfläche (12) zwischen 110° und 170° beträgt.

13. Verfahren nach Anspruch 11 oder 12, **dadurch gekennzeichnet, dass** die Verstellmittel (13) des Patientensitzes (10) mechanisch durch einen Benutzer verstellt werden, um den Patientensitz (10) in die Aufnahmeposition (16) zu bringen.

14. Verfahren nach Anspruch 11 oder 12, **dadurch gekennzeichnet, dass** die Verstellmittel (13) des Patientensitzes (10) durch mindestens ein Antriebsmittel (34) angetrieben werden und mittels einer Steuerungseinheit (35) angesteuert werden, um den Patientensitz (10) in die Aufnahmeposition (16) zu bringen.
